(19) Europäisches Patentamt
European Patent Office
Office européen des brevets

(11) Publication number: **0 480 381 A1**

(12) # EUROPEAN PATENT APPLICATION

(21) Application number: **91117144.5**

(22) Date of filing: **08.10.91**

(51) Int. Cl.[5]: **C12N 15/12**, C07K 15/00, C12P 21/02, A61K 37/02, G01N 33/68

The applicant has subsequently filed a sequence listing and declared, that it includes no new matter.

(30) Priority: **08.10.90 JP 270172/90**

(43) Date of publication of application: **15.04.92 Bulletin 92/16**

(84) Designated Contracting States: **AT BE CH DE DK ES FR GB GR IT LI LU NL SE**

(71) Applicant: **NIPPON CHEMIPHAR CO., LTD.**
**2-2-3, Iwamoto-cho**
**Chiyoda-ku Tokyo(JP)**

(72) Inventor: **Sakurai, Takeshi**
**Ruminasu Higashi No. 305, 2-11-26, Higashi**
**Tsukuba-shi, Ibaraki-ken(JP)**
Inventor: **Yanigasawa, Masashi**
**Doeru-Shugakuin No. 206, Morimoto-cho 12, Yamabana**
**Sakyo-ku, Kyoto-shi, Kyoto(JP)**
Inventor: **Masaki, Tomoh**
**547-11, Nosho-cho, Fushimi-ku**
**Kyoto-shi, Kyoto(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Endothelin receptors.**

(57) The amino acid sequence of an endothelin receptor is now analyzed. A DNA sequence encoding the receptor, a recombinant expression vector containing the DNA sequence, a process for production of the receptor, a protein composition containing the receptor and an agent for detecting the endothelin are disclosed.

EP 0 480 381 A1

## FIELD OF THE INVENTION

The present invention relates to mammalian vasoconstrictor receptors, and in more detail relates to endothelin receptors.

## BACKGROUND OF THE INVENTION

Endothelin is a strong vasoconstrictor peptide, which consists of 21 amino acids.

Endothelin has a function of constricting various arteries such as coronary artery and pulmonary artery. The vasoconstrictive function for coronary artery is extremely strong and durable. Its $EC_{50}$ is about $4 \times 10^{-10}$ M. It has also been found that endothelin is *de novo* synthesized in endothelial cells in a similar manner to synthesis of various neuropeptides and peptide hormones. As the results of these investigations, endothelin is now considered as a new intrinsic factor of a regulatory mechanism for the mammalian cardiovascular system. For example, it is considered that a large amount of endothelin released from a hypertensive or a high sensitivity to endothelin is a cause of hypertension. It is also considered that a small amount of endothelin released from a hypotensive or a low sensitivity to endothelin is a cause of hypotension.

Endothelin has recently been isolated and purified from the conditioned medium of cultured porcine aortic endothelial cells (M. Yanagisawa et al., *Nature*, 332, 411, 1988). The following structure of endothelin is determined.

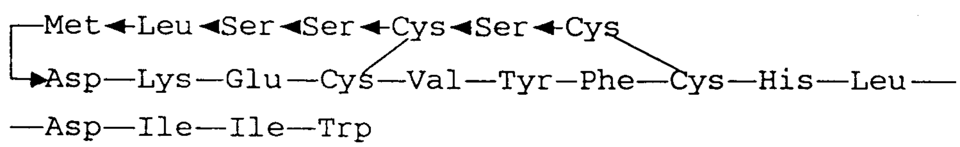

Further, it has also been found that human endothelin has the same amino acid sequence as that of porcine endothelin (Y. Itoh et al., *FEBS Lett.*, 231, 440, 1988).

Furthermore, the three isopeptides of endothelin are noted. These are reported as endothelin-1 having the above amino acid sequence, endothelin-2 ($Trp^6$, $Leu^7$) and endothelin-3 ($Thr^2$, $Phe^4$, $Thr^5$, $Tyr^6$, $Lys^7$, $Tyr^{14}$) (A. Inoue et al., *Proc. Natl. Acad. Sci. U.S.A.*, 86, 2863, 1989).

A known snake venom such as sarafotoxin-S6b (C. Takasaki et al., *Toxicon* 26, 543, 1988) has a similar structure to that of endothelin. Accordingly, this is considered as an analogue of endothelin.

The isopeptides of endothelin express their functions via a specific receptor on cell membrane. The mammalian endothelin receptor includes at least two subtypes. The subtypes are different in potency rank order with respect to affinity for the endothelin isopeptides (A. Inoue et al., *Proc, Natl. Acad. Sci. U.S.A.*, 86, 2863, 1989; T.D. Warner et al., *Eur. J. Pharmacol.*, 159, 325, 1989).

However, the structure of the endothelin receptor, namely the amino acid sequence has not yet been elucidated.

## SUMMARY OF THE INVENTION

An object of the present invention is to elucidate the structure of the endothelin receptor and to thereby provide a DNA sequence encoding the receptor.

Another object of the invention is to provide a process for production of an endothelin receptor or an analogue thereof.

A further object of the invention is to provide a protein composition containing an endothelin receptor or an analogue thereof.

According to study of the present inventors, the structure of the endothelin receptor is now elucidated. The amino acid sequence of the endothelin receptor is set forth in Figs. 1A and 1B.

There is provided by the present invention a DNA sequence substantially encoding a mammalian endothelin receptor.

The DNA sequence can be cDNA clones derived from the open reading frame of a native mammalian endothelin receptor gene. The DNA sequence can also be a sequence which is capable of hybridization to the above-mentioned cDNA clones and encodes a biologically active endothelin receptor protein. Further, the sequence can be degenerate as a result of the genetic code to the above-mentioned DNA sequences. The degenerate encodes the biologically active endothelin receptor protein.

The DNA sequence of the present invention can be integrated into a expression vector to obtain a recombinant expression vector. Therefore, the present invention further provides a process for production of a mammalian endothelin receptor or an analogue thereof, which comprises inserting the recombinant expression vector into a host cell and culturing the cell under conditions promoting expression.

The invention furthermore provides a protein composition containing a biologically active mammalian endothelin receptor or an analogue thereof which is produced as mentioned above.

The obtained protein composition containing a biologically active mammalian endothelin receptor or an analogue thereof is effective in an assay of the mammalian endothelin. The composition is also available in preparation of an antibody to the endothelin receptor. The antibody can be used in diagnosis.

It is apparent from the above-described biological activities of endothelin and the receptor thereof that an agent containing an antibody to the endothelin receptor is effective in diagnosis of hypertension or hypotension.

## BRIEF DESCRIPTION OF THE DRAWINGS

Figs. 1A and 1B show the amino acid sequence of the endothelin receptor.

Figs. 2A to 2C show the DNA sequence and the deduced amino acid sequence of the endothelin receptor.

Fig. 3 schematically illustrates pCDM8.

Fig. 4 is a graph showing a biological activity of the endothelin receptor with respect to production of inositol phosphates.

Fig. 5 is a graph showing a biological activity of the endothelin receptor with respect to increase of calcium concentration in cytoplasm.

Fig. 6 is a graph showing a biological activity of the endothelin receptor with respect to the endothelin binding characteristics.

Fig. 7 schematically illustrates an expression plasmid of the endothelin receptor.

## DETAILED DESCRIPTION OF THE INVENTION

In the present specification, the term "endothelin receptor" means proteins which are capable of binding endothelin molecules and, in their native configuration as mammalian plasma membrane proteins, presumably play a role in transducing the signal provided by endothelin to a cell. In the specification, the term includes analogs of native proteins with endothelin-binding or signal transducing activity.

The term "subtype of endothelin receptor" means molecules of endothelin receptor which show different pharmacological potency rank orders, namely different affinities or selectivities for isopeptides of endothelin or sarafotoxin.

The term "substantially (or essentially)" used in the expression "a DNA sequence substantially encoding a mammalian endothelin receptor" or the like means that a particular subject sequence, for example, a mutant sequence, varies from a reference sequence by one or more substitutions, deletions, or additions, the net effect of which does not result in an adverse functional dissimilarity between reference and subject sequences. In more detail, a sequence having at least 30 % similarity is considered to be substantially identical. The similarity preferably is at least 50 %, and more preferably is at least 80 %. For purposes of determining similarity, truncation or internal deletions of the reference sequence should be disregarded. Sequences having lesser degrees of similarity, comparable biological activity, and equivalent expression characteristics are considered to be substantial (or essential) equivalents.

The term "biologically active" used as a characteristic of endothelin receptors means either that a particular molecule has sufficient amino acid sequence similarity with the embodiments of the present invention, or that a particular molecule has sufficient amino acid sequences similarity to be capable of transmitting an endothelin stimulus to a cell as a component of a hybrid receptor construct. In more detail, the affinity (dissociation constant) of a particular molecule for endothelin-1, 2 or 3 is not more than 1 $\mu$M. In the present invention, the affinity preferably is not more than 0.1 $\mu$M, and more preferably is not more than 0.01 $\mu$M.

The term "biologically active" also means that a particular molecule has a function of accelerating production of inositol-1,4,5-triphosphate to increase calcium ion concentration in cytoplasm.

The term "DNA sequence" means a DNA polymer, in the form of a separate fragment or as a component of a larger DNA construct. The DNA construct is derived from DNA isolated at least once in substantially pure form (free of contaminating endogenous materials) and in a quantity or concentration enabling identification, manipulation, and recovery of the sequence and its component nucleotides sequences by standard biochemical methods, for example, using a cloning vector. The DNA sequences are preferably provided in the form of an open reading frame uninterrupted by internal nontranslated sequences, or introns, which are typically present in cukaryotic genes. However, genomic DNA containing the relevant sequences can also be used. Sequences of non-translated DNA may be present 5, or 3' from the open reading frame. The non-translated DNA does not interfere with manipulation or expression of the coding regions.

The term "recombinant expression vector" means a plasmid comprising a transcriptional unit. The unit comprises (a) a genetic element or elements having a regulatory role in gene expression, for example, promoters or enhancers, (b) a structural or coding sequence which is transcribed into mRNA and translated into protein, and (c) appropriate transcription and translation initiation and termination sequences. Structural elements used in yeast expression systems preferably include a leader sequence enabling extracellular secretion of translated protein by a host cell. In the case that a recombinant protein is expressed without a leader or transport sequence, it may include an N-terminal methionine residue. This residue may optionally be cleaved from the expressed recombinant protein to provide a final product.

Next, isolation of cDNA encoding the endothelin receptor and determination of the DNA sequence are described below.

A cDNA library was prepared by a reverse transcription of poly(A)$^+$RNA, which was isolated from rat lung. The DNA sequence encoding the rat endothelin receptor was isolated from the cDNA library. The library was screened by direct expression of mRNA from DNA fragments accumulated in monkey COS-7 cells using a mammalian expression vector (pCDM8). The vector contains regulatory sequences derived from SV40, polyoma virus and cytomegalovirus. The cells expressing a biologically active endothelin receptor was identified by incubating transfected COS-7 cells in a culture containing [125I-Tyr13]endothelin-1, washing the cells to remove the free labelled endothelin-1 from the cells, and placing an X-ray film on monolayers of the cells to detect binding of endothelin-1. The detected transfected cells are recognized as black spots on the film.

According to the above-mentioned method, about 20,000 cDNA clones were screened in about 40 pools to detect a black spot showing binding of endothelin-1 by an assay with respect to one pool of transfected cells. Lyophilized bacteria derive from the positive pool were cultivated in a medium. With respect to a single colony formed on an agar medium by the bacteria, screening was repeated to identify a clone which synthesizes a surface protein having a detectable endothelin-1 binding characteristic. The clone was isolated, and the sequence of insertion fragments was analyzed to determine the cDNA sequence of the rat endothelin receptor.

COS-7 cells were transfected with isolated cDNA clone to express the gene. As a result, the cells obtained a specific binding activity to endothelin-1, 2 and 3 and sarafotoxin S6b and S6c. Stimulation by endothelin was transmitted into cells as production of inositol 1,4,5-triphosphate and increase of calcium concentration in cytoplasm. In this experiment, the cells showed similar affinities for endothelin-1 and endothelin-3. In other words, the ratio of the affinity for endothelin-1 to that for endothelin-3 was in the range of 1:10 to 10:1. Therefore, it seems that this cDNA encodes one of the predicted subtypes of an endothelin receptor, which is non-specific to endothelin-1 and endothelin-3.

The above-determined DNA sequence encoding the endothelin receptor and the deduced amino acid sequence are set forth in Figs. 2A to 2C.

The DNA sequence and the amino acid sequence are described below.

There is an open reading frame of 1323b encoding 441 amino acid residues from the first ATG (initiation codon encoding methionine) to the stop codon of TGA (1324 - 1326). The 3' nontranslated region in the mRNA of the endothelin receptor encoded by this cDNA contains AUUUA sequence which destabilizes mRNA. This is analogous to transiently expressed cytokine or growth factor. The polypeptide starts from a signal sequence of 26 amino acid residues, which is predicted by von Heijine's algorithm (von Heijine, *Nucleic Acids Res*., 14, 4683, 1989). The polypeptide consists of 415 amino acid residues, and the molecular weight of 46,901 is calculated.

The encoded polypeptide contains seven stretches of 24-28 hydrophobic amino acid residues, which are likely to represent transmembrane domains. The N-terminal region preceding the first putative transmembrane domain contains two potential N-glycosylation sites. These are common characteristics of receptors such as photoreceptor rhodopsin and other G protein-coupled receptors. The amino acid sequences which are likely to represent the transmembrane domains and the first and second extracellular domains are well preserved compared with the other G protein-coupled receptor superfamily. The third intracellular domain and the C-terminal region contains serine or threonine rich sites near lysine or arginine, which is similar to the other many G protein-coupled receptors. These sites may be phosphorylated by serine/threonine kinases. Other notable characteristics of the endothelin receptor include the relatively long intracellular N-terminal portion (74 residues), the relatively short third intracellular portion (31 residues), the absence of an aspartate residue in the third transmembrane domain and the N-terminal signal sequence. These characteristics are partially analogous to those of the thyrotropin receptor, the LH-CG receptor and the tachykinin receptor.

The present invention provides the above-described DNA sequence encoding the mammalian endothelin receptor. The endothelin receptor DNA is preferably provided in a form which is capable of being expressed in a recombinant transcriptional unit under the control of mammalian, microbial, viral transcriptional or translational control elements. For example, a sequence to be expressed in a microorganism will contain no introns. In a preferred embodiment, the DNA sequence comprises at least one, but optionally more than one sequence component derived from a cDNA sequence or copy thereof.

The DNA sequences may be linked or flanked by DNA sequence prepared by assembly of synthetic oligonucleotides. However, synthetic genes assembled exclusively from oligonucleotides could be constructed using the sequence information provided herein. A representative sequence contains those substantially (essentially) identical to the nucleotide sequences shown in Figs. 2A to 2C. The coding sequences may include codons encoding one or more additional amino acids located at the N-terminus, for example, an N-terminal ATG codons specifying methionine linked with reading frame in the nucleotide sequence. Due to code degeneracy, there can be considerable variation in nucleotide sequences encoding the same amino acid sequence. Other embodiments include sequences capable of hybridizing to the representative sequence under moderately stringent conditions (42 °C, 20 % (v/v) formamide). The other sequences degenerate to those described above which encode biologically active endothelin receptor polypeptides.

The present invention also provides expression vectors for producing useful quantities of purified endothelin receptor. The vectors can comprise synthetic or cDNA derived DNA fragments encoding mammalian endothelin receptors or bioequivalent homologues operably linked to regulatory elements derived from mammalian, bacterial, yeast, bacteriophage or viral genes. Useful regulatory elements are described in greater detail below. Following transformation, transfection or infection of appropriate cell lines, such vectors can be induced to express recombinant protein.

Mammalian endothelin receptors can be expressed in mammalian cells, yeast, bacteria, or other cells under the control of appropriate promoters. Cell-free translation systems could also be employed to produce mammalian endothelin receptor using mRNAs derived from the DNA constructs of the present invention. Appropriate cloning and expression vectors for use with bacterial, fungal, yeast, and mammalian cellular hosts are described by Pouwels et al. (*Cloning Vectors: A Laboratory Manual*, Elsevier, New York, 1985), the relevant disclosure of which is hereby incorporated by reference.

Various mammalian cell culture systems can be employed to express recombinant protein. Examples of suitable mammalian host cell lines include the COS-7 lines of monkey kidney cells, described by Gluzman (*Cell* 23:175, 1981), and other cell lines capable of expressing an appropriate vector, for example, C127, 3T3, CHO, HeLa and BHK cell lines. Mammalian expression vectors may comprise nontranscribed elements such as an origin of replication, a suitable promoter and enhancer, and other 5' or 3' flanking nontranscribed sequences, and 5' or 3' nontranslated sequences, such as necessary ribosome binding sites, a polyadenylation site, splice donor and acceptor sites, and termination sequences. DNA sequences derived from the SV40 viral genome, for example, SV40 replication origin, early promoter, enhancer, splice, and polyadenylation sites, may be used to provide the other genetic elements required for expression of a heterologous DNA sequence. Additional details regarding the use of a mammalian high expression vector to produce a recombinant mammalian endothelin receptor are provided in Examples 4 and 5, below. Exemplary vectors can be constructed as disclosed by Okayama and Berg (*Mol Cell Biol.* 3, 280, 1983).

A useful system for stable high level expression of mammalian receptor cDNAs in C127 rat mammary epithelial cells can be constructed substantially as described by Cosman et al. (*Molecular Immunol.* 23:935, 1986).

Yeast systems, preferably employing Saccharomyces species such as *S. cerevisiae*, can also be employed for expression of the recombinant proteins of the present invention. Yeast of other genera, for example, Pichis or Kluyveromyces, have also been employed as production strains for recombinant proteins.

Generally, useful yeast vectors will include origins of replication and selectable markers permitting transformation of both yeast and *E. coli*, e.g., the ampicillin resistance gene ($Amp^r$) of *E. coli* and *S. cerevisiae* TRP1 gene, and a promoter derived from a highly-expressed yeast gene to induce transcription of a downstream structural gene. Such promoters can be derived from yeast transcriptional units encoding highly expressed genes such as 3-phosphoglycerate kinase (PGK), α-factor, acid phosphatase, or heat shock proteins, among others. The heterologous structural sequence is assembled in appropriate reading frame with translation initiation and termination sequences, and, preferably, a leader sequence capable of directing secretion of translated protein into the extracellular medium. Optionally, the heterologous sequences can encode a fusion protein including an N-terminal identification peptide or other sequence imparting desired characteristics, e.g., stabilization or simplified purification of expressed recombinant product.

Useful yeast vectors can be assembled using DNA sequences from pBR322 ($Amp^r$ gene and origin of replication) for selection and replication in *E. coli* and yeast DNA sequences including a glucose-repressible alcohol dehydrogenase 2 (ADH2) promoter. The ADH2 promoter has been described by Russell et al. (*J. Biol. Chem*. 258:2674, 1982) and Beier et al. (*Nature* 300:724, 1982). Such vectors may also include a yeast TRP1 gene as a selectable marker and the yeast 2 μ origin of replication. A yeast leader sequence, for example, the α-factor leader which directs secretion of heterologous proteins from a yeast host, can be inserted between the promoter and the structural gene to be expressed (see Kurian et al., U.S. Patent No. 4,546,082; Kurian et al., *Cell* 30:933, 1982); and Bittner et al., *Proc. Natl. Acad. Sci. USA* 81:983 1984).

The leader sequence may be modified to contain, near its 3' end, one or more useful restriction sites to facilitate fusion of the leader sequence to foreign genes.

Suitable yeast transformation protocols are known to those skilled in the art; an exemplary technique is described by Hinnen et al. (*Proc. Natl. Acad. Sci. USA* 75:1929, 1978), selecting for $Trp^+$ transformants in a selective medium consisting of 0.67 % yeast nitrogen source, 0.5 % casamino acids, 2 % glucose, 10 μg/ml adenine and 20 μg/ml uracil.

Host strains transformed by vectors comprising the ADH2 promoter may be grown for expression in a rich medium consisting of 1 % yeast extract, 2 % peptone and 1 % glucose supplemented with 80 μg/ml adenine and 80 μg/ml uracil. Deregulation of the ADH2 promoter occurs upon exhaustion of medium glucose. Crude yeast supernatants are harvested by filtration and held at 4 ° C prior to further purification.

Useful expression vectors for bacterial use are constructed by inserting a DNA sequence encoding mammalian endothelin receptor together with suitable translation initiation and termination signals in operable reading frame with a functional promoter. The vector will comprise one or more phenotypic selectable markers and an origin of replication to ensure growth within the host. Suitable prokaryotic hosts for transformation include *E. coli*, *Bacillus Subtilis, Salmonella typhimurium* and various species within the genera Pseudomonas, Streptomyces, and Staphylococcus, although others may also be employed as a matter of choice.

Expression vectors are conveniently constructed by cleavage of cDNA clones at sites close to the codon encoding the N-terminal residue of the mature protein. Synthetic oligonucleotides can then be used to "add back" any deleted sections of the coding region and to provide a linking sequence for ligation of the coding fragment in appropriate reading frame in the expression vector, and optionally a codon specifying an initiator methionine.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example pKK223-3p (Pharmacia Fine Chemicals, Uppsala, Sweden) and pGEM1 (Projema Biotec, Madison, WI, USA). These pBR322 "main chain" sections are combined with an appropriate promoter and the structural sequence to be expressed.

A particularly useful bacterial expression system employs the phage λ $P_L$ promoter and cI857 thermolabile repressor. Plasmid vectors available from the American Type Culture Collection which incorporate derivatives of the λ $P_L$ promoter include plasmid pHUB2, resident in *E. coli* strain JMB9 (ATCC37092) and pPLc28, resident in *E. coli* RR1 (ATCC53082). Other useful promoters for expression in *E. coli* include the T7 RNA polymerase promoter described by Studier et al. (*J. Mol. Biol*. 189:113, 1986), the *lacZ* promoter described by Laner (*J. Mol. Appl. Genet*. 1:139-147, 1981) which is available as ATCC 37121, and the tac promoter described by Maniatis (*Molecular Cloning: A Laboratory Manual*, Cold Spring Harbor Laboratory, 1982, p412) which is available as ATCC37138.

Following transformation of a suitable host strain and growth of the host strain to an appropriate cell density, the selected promoter is derepressed by appropriate means (e.g., temperature shift or chemical induction) and cells cultured for an additional period. Cells are typically harvested by centrifugation, disrupted by physical or chemical means, and the resulting crude extract retained for further purification. Cells are grown, for example, in a 10 liter fermenter employing conditions of maximum aeration and vigorous agitation. An antifoaming agent (Antifoam A) is preferably employed. Cultures are grown at 30 ° C in the superinduction medium disclosed by Mott et al. (*Proc. Natl. Acad. Sci. USA* 82:88, 1985), alternatively including antibiotics, derepressed at a cell density corresponding to $A_{600}$ = 0.4-0.5 by elevating the temperature to 42 ° C, and harvested for 2-20 hours, preferably 3-6 hours after the upward temperature shift. The cell mass is initially concentrated by filtration or other means, then centrifuged at 10,000 × g (10,000 G) for 10 minutes at 4 °C, followed by rapidly freezing the cell pellet.

Preferably, purified mammalian endothelin receptors or bioequivalent analogs are prepared by culturing suitable host/vector systems to express the recombinant translation products of the synthetic genes of the present invention, which are then purified from culture media.

An alternative process for producing purified endothelin receptor involves purification from cell culture supernatants or extracts. In this approach, a cell line which elaborates useful quantities of the protein is employed. Supernatants from such cell lines can be optionally concentrated using a commercially available protein concentration filter, for example, an Amicon or Millipore Falcon ultra-filtration unit. Following the concentration step, the concentrate can be applied to a suitable purification matrix as previously described. For example, a suitable affinity matrix can comprise an endothelin receptor or lectin or antibody molecule bound to a suitable support. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymetyl groups.

Finally, one or more reversed-phase high performance liquid chromatography (RP-HPLC) steps employing hydrophobic RP-HPLC media, e.g., silica gel having methyl or other aliphatic groups, can be employed to further purify an endothelin receptor composition. Some or all of the foregoing purification steps, in various combinations, can also be employed to provide a homogeneous recombinant protein.

Recombinant protein produced in bacterial culture is usually isolated by initial extraction from cell pellets, followed by one or more concentration, salting-out, aqueous ion exchange or gel filtration chromatography steps. Finally, high performance liquid chromatography (HPLC) can be employed for final purification steps. Microbial cells employed in expression of recombinant mammalian endothelin receptor can be disrupted by any convenient method, including freeze-thaw cycling, sonication, mechanical disruption or use of cell lysing agents.

Fermentation of yeast which expresses mammalian endothelin receptor as a secreted protein greatly simplifies purification. Secreted recombinant protein resulting from a large-scale fermentation can be purified by methods analogous to those disclosed by Urdal et al. (*J. Chromatog.* 296:171, 1984). This reference describes two sequential, reversed-phase HPLC steps for purification of recombinant human GM-CSF on a preparative HPLC column.

In its various embodiments, the present invention provides substantially (essentially) homogeneous recombinant mammalian endothelin receptor polypeptides free of contaminating endogenous material.

Recombinant endothelin receptor proteins of the present invention also include suitable peptide or protein sequences employed as aids to expression in microorganisms or purification of microbially expressed proteins.

Bioequivalent analogues of the proteins of this invention include various analogs, for example, truncated versions of endothelin receptors wherein terminal residues or sequences, which exists in internal cell and are not needed for biological activity, are deleted.

Examples of the present invention are described below.

EXAMPLE 1

A cDNA library was constructed by a reverse transcription of poly(A)$^+$RNA according to a procedure similar to that of Chirgwin et al. (*Biochem.*, 18, 5294, 1979). The poly(A)$^+$RNA was isolated from total RNA extracted from rat lung. In more detail, the tissue was dissolved in a solution of guanidinium isothiocyanate. The solution was layered over a pad of CsCl, and was centrifuged to precipitate RNA. The RNA pellet was resuspended and further purified by protease digestion, organic extraction and alcohol precipitation. Poly-(A$^+$)RNA was isolated by oligo dT cellulose chromatography and double-stranded cDNA was prepared by a method similar to that of Gubler and Hoffman (*Gene* 25, 263, 1983). Briefly, the RNA was copied into cDNA by reverse transcriptase using either oligo dT or random oligonucleotides as primer. The cDNA was made double-stranded by incubation with *E. coli* DNA polymerase I and RNase H, and the ends made flush by further incubation with $T_4$ DNA polymerase. BstXI linker was added to the blunt-ended cDNA, and then short chains were removed by a gel filtration chromatography using Sepharose CL-2B. The cDNA was combined with a high expression plasmid vector for mammalian cells (pCDM8). A schematic illustration of pCDM8 is shown in Fig. 3.

The pCDM8 vector is a plasmid vector of 4.8 kb which contains a replication initiating point of SV 40 and polyoma and another replication initiating point of cytomegalo-virus/T7RNA polymerase promoter and M13 (see Seed, *Nature*, 329, 840, 1987).

The resulting rat lung cDNA library on pCDM8 was used to transform *E. coli* (Mc 1061/$P_3$) to provide about 5 × $10^5$ colonies. These recombinants were preserved in pools for each 500 colonies. The pooled DNA was used to transfect a sub-confluent layer of monkey COS-7 cells using DEAE-dextran followed by chloroquine treatment, as described by Seed et al., *Proc. Natl. Acad. Sci. USA*. 84, 3365, 1987). The cells were then grown in culture for three days to permit transient expression of the inserted sequences. The cell monolayers in each plate were assayed for endothelin receptor binding as follows. To each plate was added 2 ml of DMEM medium of 3 % BSA containing 2.5 × $10^{-11}$ M [$^{125}$I-Tyr$^{13}$]endothelin-1, and the plates were incubated for 2 hours at 37 °C at 5 % $CO_2$. This medium was then discarded, and each plate was once washed with the above medium (containing no [$^{125}$I-Tyr$^{13}$]endothelin-1), and twice washed with PBS (pH 7.4). Each plate was then fixed with PBS containing 2.5 % glutaraldehyde. Each plate was dried in the air. The edges of each plate were broken off, leaving a flat disk which was contacted with a X-ray film for 8 to 72 hours at -80 °C. The endothelin-1 binding activity was visualized on the exposed films as a black spot. About 2 × $10^4$ recombinants were screened from the library as is mentioned above. Thus COS-7 recombinants transfected with a single clone which is capable of inducing expression of endotherin receptor were obtained.

The inserted fragments of clone prETR-7 were subcloned to pUC118/119 plasmid, and its DNA sequence was determined according to the dideoxy method (see Sanger et al., *Proc. Natl. Acad. Sci. USA*, 74, 5463, 1977).

## EXAMPLE 2

## Characteristics of rat endothelin receptor expressed on COS-7 cells (information transport system from receptor)

### i) Production of inositol phosphates

The COS-7 cells transfected with prETR-7 were cultivated for 3 days, and were detached using 0.025 % trypsin/0.05 % EGTA. The reaction was stopped by soybean trypsin inhibitor, and the cells were collected by centrifugation. The cells were washed twice with a solution A (140 mM sodium chloride, 4 mM potassium chloride, 1 mM disodium phosphate, 1 mM magnesium chloride, 1.25 calcium chloride, 11 mM glucose, 5 mM HEPES (pH 7.4), 0.2 % BSA), and incubated for 3 hours in the solution A further containing 80 μCi/ml $^3$H-myo-inositol. The cells were then stimulated with $10^{-7}$ M endothelin-1 or endothelin-3 for 30 minutes in the presence of 10 mM lithium chloride. The inositol phosphates were separated by AG-1X8 anion exchange chromatography, and were quantified by measuring radio activity.

The results are set forth in Fig. 4.

### ii) Increase of calcium concentration in cytoplasm

The collected and washed COS-7 cells were incubated at 20 °C for 60 minutes in the solution A further containing 4 μmfura-2/AM. The cells were twice washed and preserved at 20 °C in the solution A (containing no fura-2/AM). For one experiment, about $10^6$ cells in 1 ml were used. The cells were continuously stirred in cubet, and measured at excitation spectrum of 340 nm or 380 nm and at fluorescence spectrum of 500 nm. Endothelin-1, 2 or 3 of various concentration was injected by a microsyringe through a rubber septum. The calcium concentration was calculated from the ratio of the fluorescence strength according to the method of Grynkiewicz et al. (*J. Biol. Chem.*, 260, 3440, 1985).

EC50 of the endothelin receptor expressed on COS-7 cells were about 2 × $10^{-11}$ M. The effects were analogous with respect to endothelin-1, 2 and 3.

The results are set forth in Fig. 5.

### iii) Binding experiment of [$^{125}$I-Tyr$^{13}$]endothelin-1

The COS-7 cells transfected with prETR-7 were cultivated in 12 holes plate, and washed once with the solution A. The cells were incubated at 37 °C for 60 minutes in 1 ml solution A containing [$^{125}$I-Tyr$^{13}$]-endothelin-1 and endothelin-1, 2 or 3 of various concentration. The cells were well washed, and the radio activities binding the cell were measured.

The binding affinity of the endothelin receptor expressed on COS-7 cells was about 2 × $10^{-9}$ M. The affinities were analogous with respect to endothelin-1, 2 and 3.

The results are set forth in Fig. 6.

EXAMPLE 3

Expression in tissues by northern blots using endothelin receptor cDNA

Poly(A)$^+$RNA was extracted from various rat tissues in the same manner as is mentioned above. Each 10 $\mu$g of the RNAs was separated using formaldehyde/1.1 % agarose gel electrophoresis, and transferred to gene screen plus membrane (NEN, Dupont). Then, 2kb of cDNA fragments was labeled by $\alpha$-$^{32}$P-dCTP according to a random prim method to 8 $\times$ 108 c.p.m./mg, and used as a probe. Hybridization was carried out at 42 °C in a solution of 1M sodium chloride, 50 % formaldehyde, 1 % SDS and 250 $\mu$g salmon sperm DNA. The sample was washed with 2 $\times$ SSC/1 % SDS five times at 22 °C, once at 65 °C, and once washed with 0.1 $\times$ SSC/0.1 % SDS at 50 °C. Autoradiography was carried out for 10 hours.

Northern blots were carried out with respect to poly(A)$^+$RNAs extracted from 14 rat tissues. As the results, 5.0 kb of endothelin receptor mRNA was detected in the tissues in which a physiological function of endothelin had been reported. A large amount of endothelin receptor mRNA was expressed in brain, lung, kidney, heart, eyeball liver, stomach and adrenal in order of amount. A relatively large amount was expressed in womb, small intestine and glandular submandibularis, a trace amount was expressed in testis and skeletal muscle, and there was no expression in spleen and aorta smooth muscle.

EXAMPLE 4

Expression of endothelin receptor by dhfr-defective strain of CHO cell line

Endothelin receptor cDNA insert was integrated into the down stream of the promoter of an expression vector pSVD (see R.A. Poovman et al., *Proteins*, 1, 139, 1986) which contains dhfr (dihydroforate reductase) gene and a promoter derived from SV40 virus. Thus an endothelin receptor expression plasmid pSVDrETR was prepared.

The endothelin receptor expression plasmid was set forth in Fig. 7.

CHO (chinese hamster ovary) dhfr$^-$ cells (see G. Urlaub et al., *Proc. Natl. Acad. Sci. USA* 77, 4216, 1980) were cultivated as a subconfluent monolayer in a HamF12 medium containing 10 % FBS.

The CHO dhfr$^-$ cells were transfected with the endothelin receptor expression plasmid pSVDrETR according to the calcium phosphate method and glycerol treatment. After 24 hours, the transfected cells were subcultured in an area about 20 times as large as the previous area. After 24 hours, the cells were well inoculated, and the medium was replaced with DMEM medium containing 10 % dialysis FBS.

Thus only the cells transfected with dhfr gene into genome grew and their colony was formed.

The well grown colony was detached using trypsin according to the penicillin cup method, and subcultured in 12 holes plate using the same DMEM medium.

Thus isolated clonal cells expressed endothelin receptor, which was confirmed by the observation of increase of calcium concentration in cytoplasm using fura-2 when endothelin was added to the cells as described at ii) in Example 2. The expression was also confirmed by the binding experiment of [$^{125}$I-Tyr$^{13}$]-endothelin-1 as described at iii) in Example 2.

EXAMPLE 5

Expression of endothelin receptor bv L cell

Fragments inserted with human endothelin receptor cDNA was integrated into the down stream of the promoter of an expression vector pME18Sf$^-$ which contains the SRa promoter.

L cells (mouse fibroblast) were cultivated as a subconfluent monolayer in a DMEM medium containing 10 % FBS.

The L cells were stably transfected with the human endothelin receptor expression plasmid and a plasmid pSVneo containing nomycin resistant gene according to the calcium phosphate method and glycerol treatment. After 24 hours, the transfected cells were subcultured in an area about 10 times as large as the previous area. After 24 hours, the cells were well inoculated, and the medium was replaced with DMEM medium containing 10 % FBS and 0.5 mg/ml of neomycin.

Thus only the cells transfected with neomycin resistant gene into genome grew and their colony was formed. The human endothelin receptor expression plasmid is usually integrated simultaneously with the integration of the plasmid pSVneo.

The well grown colony was detached using trypsin according to the penicillin cup method, and subcultured in 12 holes plate using the same DMEM medium.

Thus isolated clonal cells expressed endothelin receptor, which was confirmed by the observation of increase of calcium concentration in cytoplasm using fura-2 when endothelin was added to the cells as described at ii) in Example 2.

The affinities of the cells transfected with the human endothelin receptor expression plasmid were analogous with respect to endothelin-1 and 3.

Specimen Sequence Listing

SEQ ID NO: 1
SEQUENCE TYPE: Protein
SEQUENCE LENGTH: 441 amino acids

ORIGINAL SOURCE
ORGANISM: rat
IMMEDIATE EXPERIMENTAL SOURCE
NAME OF CELL LINE: rat lung

PROPERTIES: endothelin receptor

```
Met Gln Ser Ser Ala Ser Arg Cys Gly Arg Ala Leu Val Ala Leu
  1               5                  10                  15

Leu Leu Ala Cys Gly Leu Leu Gly Val Trp Gly Glu Lys Arg Gly
                 20                  25                  30

Phe Pro Pro Ala Gln Ala Thr Pro Ser Leu Leu Gly Thr Lys Glu
                 35                  40                  45

Val Met Thr Pro Pro Thr Lys Thr Ser Trp Thr Arg Gly Ser Asn
                 50                  55                  60

Ser Ser Leu Met Arg Phe Arg Thr Ala Glu Val Thr Lys Gly Gly
                 65                  70                  75

Arg Val Ala Gly Val Pro Pro Arg Ser Phe Pro Pro Pro Cys Gln
                 80                  85                  90

Arg Lys Ile Glu Ile Asn Lys Thr Phe Lys Tyr Ile Asn Thr Ile
                 95                 100                 105

Val Ser Cys Leu Val Phe Val Leu Gly Ile Ile Gly Asn Ser Thr
                110                 115                 120

Leu Leu Arg Ile Ile Tyr Lys Asn Lys Cys Met Arg Asn Gly Pro
                125                 130                 135

Asn Ile Leu Ile Ala Ser Leu Ala Leu Gly Asp Leu Leu His Ile
                140                 145                 150

Ile Ile Asp Ile Pro Ile Asn Ala Tyr Lys Leu Leu Ala Gly Asp
                155                 160                 165

Trp Pro Phe Gly Ala Glu Met Cys Lys Leu Val Pro Phe Ile Gln
                170                 175                 180
```

```
Lys Ala Ser Val Gly Ile Thr Val Leu Ser Leu Cys Ala Leu Ser
                185                 190                 195

Ile Asp Arg Tyr Arg Ala Val Ala Ser Trp Ser Arg Ile Lys Gly
                200                 205                 210

Ile Gly Val Pro Lys Trp Thr Ala Val Glu Ile Val Leu Ile Trp
                215                 220                 225

Val Val Ser Val Val Leu Ala Val Pro Glu Ala Ile Gly Phe Asp
                230                 235                 240

Val Ile Thr Ser Asp Tyr Lys Gly Lys Pro Leu Arg Val Cys Met
                245                 250                 255

Leu Asn Pro Phe Gln Lys Thr Ala Phe Met Gln Phe Tyr Lys Thr
                260                 265                 270

Ala Lys Asp Trp Trp Leu Phe Ser Phe Tyr Phe Cys Leu Pro Leu
                275                 280                 285

Ala Ile Thr Ala Ile Phe Tyr Thr Leu Met Thr Cys Glu Met Leu
                290                 295                 300

Arg Lys Lys Ser Gly Met Gln Ile Ala Leu Asn Asp His Leu Lys
                305                 310                 315

Gln Arg Arg Glu Val Ala Lys Thr Val Phe Cys Leu Val Leu Val
                320                 325                 330

Phe Ala Leu Cys Trp Leu Pro Leu His Leu Ser Arg Ile Leu Lys
                335                 340                 345

Leu Thr Leu Tyr Asp Gln Ser Asn Pro Gln Arg Cys Glu Leu Leu
                350                 355                 360

Ser Phe Leu Leu Val Leu Asp Tyr Ile Gly Ile Asn Met Ala Ser
                365                 370                 375

Leu Asn Ser Cys Ile Asn Pro Ile Ala Leu Tyr Leu Val Ser Lys
                380                 385                 390

Arg Phe Lys Asn Cys Phe Lys Ser Cys Leu Cys Cys Trp Cys Gln
                395                 400                 405

Thr Phe Glu Glu Lys Gln Ser Leu Glu Glu Lys Gln Ser Cys Leu
                410                 415                 420

Lys Phe Lys Ala Asn Asp His Gly Tyr Asp Asn Phe Arg Ser Ser
                425                 430                 435

Asn Lys Tyr Ser Ser Ser
                440
```

SEQ ID NO: 2
SEQUENCE TYPE: Nucleotide with corresponding protein
SEQUENCE LENGTH: 1965 base pairs

STRANDEDNESS: double
TOPOLOGY: linear
MOLECULE TYPE: cDNA

ORIGINAL SOURCE
ORGANISM: rat
IMMEDIATE EXPERIMENTAL SOURCE
NAME OF CELL LINE: rat lung

PROPERTIES: endothelin receptor

```
GGTGGCGTGC GCCCAAGTTC CCCATTGGCG CGCAAACTTA ACTTACTGTT    50

GTGGCGCGGG TAGAGACAAC CCGGCTAGGG TGAGTGTTTT CAGAGGCGTG   100

GCTGGGTAGC TGACTAAAGT ACCCTCTCTT CATTCCCCTG TTGTTCTCCA   150

GACTGAAAAC GGCGGAGCGG CTACGGGACT CTCACAGGAG CAAGCTGCAA   200

C ATG CAA TCG TCC GCA AGC CGG TGC GGA CGC GCC TTG         237
  Met Gln Ser Ser Ala Ser Arg Cys Gly Arg Ala Leu
    1               5                   10

GTG GCG CTG CTG CTG GCC TGT GGC TTG TTG GGG GTA           273
Val Ala Leu Leu Leu Ala Cys Gly Leu Leu Gly Val
        15                  20

TGG GGA GAG AAA AGA GGA TTC CCA CCT GCC CAG GCC           309
Trp Gly Glu Lys Arg Gly Phe Pro Pro Ala Gln Ala
 25                 30                  35

ACA CCA TCT CTT CTC GGG ACT AAA GAA GTT ATG ACG           345
Thr Pro Ser Leu Leu Gly Thr Lys Glu Val Met Thr
            40                  45

CCA CCC ACT AAG ACC TCC TGG ACT AGA GGT TCC AAC           381
Pro Pro Thr Lys Thr Ser Trp Thr Arg Gly Ser Asn
     50                  55                  60

TCC AGT CTG ATG CGT TTC CGC ACT GCG GAG GTG ACC           417
Ser Ser Leu Met Arg Phe Arg Thr Ala Glu Val Thr
                65                  70
```

```
AAA GGA GGG AGG GTG GCT GGA GTC CCG CCA AGA TCC          453
Lys Gly Gly Arg Val Ala Gly Val Pro Pro Arg Ser
         75                  80

TTC CCT CCT CCG TGC CAA CGA AAA ATT GAG ATC AAC          489
Phe Pro Pro Pro Cys Gln Arg Lys Ile Glu Ile Asn
 85                 90                  95

AAG ACT TTT AAA TAC ATC AAC ACG ATT GTA TCA TGC          525
Lys Thr Phe Lys Tyr Ile Asn Thr Ile Val Ser Cys
            100                 105

CTC GTG TTC GTG CTA GGC ATC ATC GGG AAC TCC ACA          561
Leu Val Phe Val Leu Gly Ile Ile Gly Asn Ser Thr
    110                 115                 120

CTG CTA AGA ATC ATC TAC AAG AAC AAG TGC ATG AGA          597
Leu Leu Arg Ile Ile Tyr Lys Asn Lys Cys Met Arg
                125                 130

AAT GGT CCC AAT ATC TTG ATC GCC AGC CTG GCT CTG          633
Asn Gly Pro Asn Ile Leu Ile Ala Ser Leu Ala Leu
        135                 140

GGA GAT CTG CTA CAC ATC ATC ATC GAC ATT CCC ATT          669
Gly Asp Leu Leu His Ile Ile Ile Asp Ile Pro Ile
145                 150                 155

AAT GCC TAC AAG CTG CTG GCA GGG GAC TGG CCA TTT          705
Asn Ala Tyr Lys Leu Leu Ala Gly Asp Trp Pro Phe
            160                 165

GGA GCT GAG ATG TGC AAG CTG GTG CCC TTC ATA CAG          741
Gly Ala Glu Met Cys Lys Leu Val Pro Phe Ile Gln
    170                 175                 180

AAG GCT TCT GTG GGG ATC ACA GTG TTG AGT CTA TGT          777
Lys Ala Ser Val Gly Ile Thr Val Leu Ser Leu Cys
                185                 190

GCT CTA AGT ATT GAC AGA TAT CGA GCT GTT GCT TCT          813
Ala Leu Ser Ile Asp Arg Tyr Arg Ala Val Ala Ser
        195                 200

TGG AGT CGA ATT AAA GGA ATT GGG GTT CCA AAA TGG          849
Trp Ser Arg Ile Lys Gly Ile Gly Val Pro Lys Trp
205                 210                 215

ACA GCA GTA GAA ATT GTT TTA ATT TGG GTG GTC TCT          885
Thr Ala Val Glu Ile Val Leu Ile Trp Val Val Ser
            220                 225

GTG GTT CTG GCT GTC CCT GAA GCC ATA GGT TTT GAT          921
Val Val Leu Ala Val Pro Glu Ala Ile Gly Phe Asp
    230                 235                 240
```

```
GTG ATT ACG TCG GAC TAC AAA GGA AAG CCC CTA AGG          957
Val Ile Thr Ser Asp Tyr Lys Gly Lys Pro Leu Arg
                245                 250

GTC TGC ATG CTT AAT CCC TTT CAG AAA ACA GCC TTC          993
Val Cys Met Leu Asn Pro Phe Gln Lys Thr Ala Phe
        255                 260

ATG CAG TTT TAC AAG ACA GCC AAA GAC TGG TGG CTG         1029
Met Gln Phe Tyr Lys Thr Ala Lys Asp Trp Trp Leu
265                 270                 275

TTC AGT TTC TAC TTC TGC TTG CCG CTA GCC ATC ACT         1065
Phe Ser Phe Tyr Phe Cys Leu Pro Leu Ala Ile Thr
            280                 285

GCG ATC TTT TAC ACC CTA ATG ACC TGT GAG ATG CTC         1101
Ala Ile Phe Tyr Thr Leu Met Thr Cys Glu Met Leu
    290                 295                 300

AGA AAG AAA AGT GGT ATG CAG ATT GCC TTG AAT GAC         1137
Arg Lys Lys Ser Gly Met Gln Ile Ala Leu Asn Asp
                305                 310

CAC TTA AAG CAG AGA CGA GAA GTG GCC AAG ACA GTA         1173
His Leu Lys Gln Arg Arg Glu Val Ala Lys Thr Val
        315                 320

TTC TGC CTG GTC CTC GTG TTT GCC CTC TGT TGG CTT         1209
Phe Cys Leu Val Leu Val Phe Ala Leu Cys Trp Leu
325                 330                 335

CCC CTT CAC CTC AGC AGG ATT CTG AAG CTC ACC CTT         1245
Pro Leu His Leu Ser Arg Ile Leu Lys Leu Thr Leu
            340                 345

TAT GAC CAG AGC AAT CCT CAG AGG TGT GAA CTT CTG         1281
Tyr Asp Gln Ser Asn Pro Gln Arg Cys Glu Leu Leu
    350                 355                 360

AGT TTT TTG CTG GTT TTG GAC TAC ATT GGT ATC AAC         1317
Ser Phe Leu Leu Val Leu Asp Tyr Ile Gly Ile Asn
                365                 370

ATG GCT TCT TTG AAT TCC TGC ATT AAT CCA ATC GCT         1353
Met Ala Ser Leu Asn Ser Cys Ile Asn Pro Ile Ala
        375                 380

CTG TAT TTG GTG AGC AAG AGA TTC AAA AAC TGC TTT         1389
Leu Tyr Leu Val Ser Lys Arg Phe Lys Asn Cys Phe
385                 390                 395
```

```
AAG TCG TGT TTG TGC TGC TGG TGC CAA ACG TTT GAG         1425
Lys Ser Cys Leu Cys Cys Trp Cys Gln Thr Phe Glu
            400                 405

GAA AAA CAG TCC TTA GAG GAG AAG CAA TCC TGC TTG         1461
Glu Lys Gln Ser Leu Glu Glu Lys Gln Ser Cys Leu
    410                 415                 420

AAG TTC AAA GCT AAC GAT CAC GGA TAC GAC AAC TTC         1497
Lys Phe Lys Ala Asn Asp His Gly Tyr Asp Asn Phe
                425                 430

CGC TCC AGC AAT AAA TAC AGC TCA TCT                     1524
Arg Ser Ser Asn Lys Tyr Ser Ser Ser
        435                 440

TGAAGGAAGG AACACTCACT GAATCTCATT GTCCTCATCG TGGACAGATA 1574

GCATTAAAAC AAAATGAAAC CTTTGCCAAA CCCAAACGGA AAACCGTGCT 1624

TGCGGAAAGG TGTGCACGCA TGGGAGAGGG ATTGTTTTTT AACCGTTCTA 1674

ACTTTCCACA CCTGATATTT CACGGGCTGT TTACAACCTA AGAAAGCCAT 1724

GGGAATGAAT GAAGCCTCGG GAAAGCACTT AGATTCTTAG TCAAGCACTT 1774

CAGCACGGCT CTTAAAAGCC CTCACTGCAC TCACAGCCCA CTTACATTTA 1824

AAAACAAGAA CTCAAACTCT ATTCAGGGGT TTATTATCCA GTCCTATGAA 1874

TCTGGATACA GGAATGCATG ACATTGCAAA ACAATTCTTA AAGCAAAGTT 1924

TCAATTGCTC GATTTGAGAC AAAAAACAAA ACAAAAAAAA A          1965
```

## Claims

1. A DNA sequence substantially encoding a mammalian endothelin receptor.

2. The DNA sequence as claimed in claim 1, wherein the mammalian endothelin receptor has such a pharmacological activity that the ratio of the affinity of the receptor for endothelin-1 to that for endothelin-3 is in the range of 1:10 to 10:1.

3. The DNA sequence as claimed in claim 1, wherein the sequence comprises cDNA clones having a nucleotide sequence derived from the coding region of a native mammalian endothelin receptor gene.

4. The DNA sequence as claimed in claim 1, wherein the sequence is capable of hybridization to cDNA clones under moderately stringent conditions and encodes a biologically active endothelin receptor protein, said cDNA clones having a nucleotide sequence derived from the coding region of a native mammalian endothelin receptor gene.

5. The DNA sequence as claimed in claim 1, wherein the sequence is degenerate as a result of the genetic code to cDNA clones and encodes a biologically active endothelin receptor protein, said cDNA clones having a nucleotide sequence derived from the coding region of a native mammalian endothelin receptor gene.

6. The DNA sequence as claimed in claim 1, wherein the sequence is degenerate as a result of the genetic code to a sequence capable of hybridization to cDNA clones under moderately stringent conditions and encodes a biologically active endothelin receptor protein, said cDNA clones having a nucleotide sequence derived from the coding region of a native mammalian endothelin receptor gene.

**7.** The DNA sequence as claimed in claim 1, wherein the sequence consists essentially of a synthetic gene which encodes a mammalian endothelin receptor protein which is capable of being expressed in a recombinant transcriptional unit comprising inducible regulatory elements derived from a microbial or viral operon.

**8.** The DNA sequence as claimed in claim 1, wherein the sequence has at least 30 % similarity to a DNA sequence encoding a mammalian endothelin receptor.

**9.** The DNA sequence as claimed in claim 1, wherein the sequence has at least 50 % similarity to a DNA sequence encoding a mammalian endothelin receptor.

**10.** The DNA sequence as claimed in claim 1, wherein the sequence has at least 80 % similarity to a DNA sequence encoding a mammalian endothelin receptor.

**11.** A recombinant expression vector which contains a DNA sequence substantially encoding a mammalian endothelin receptor.

**12.** A process for production of a mammalian endothelin receptor or an analogue thereof, which comprises transfecting a host cell with a recombinant expression vector and culturing the cell under conditions promoting expression, said vector containing a DNA sequence substantially encoding a mammalian endothelin receptor.

**13.** A protein composition containing a biologically active mammalian endothelin receptor or an analogue thereof which is produced by a recombinant cell culture.

**14.** An agent for detecting a mammalian endothelin, wherein the agent contains a biologically active mammalian endothelin receptor or an analogue thereof.

# FIG. 1A

Met-Gln-Ser-Ser-Ala-Ser-Arg-Cys-Gly-Arg-Ala-
Leu-Val-Ala-Leu-Leu-Leu-Ala-Cys-Gly-Leu-Leu-
Gly-Val-Trp-Gly-Glu-Lys-Arg-Gly-Phe-Pro-Pro-
Ala-Gln-Ala-Thr-Pro-Ser-Leu-Leu-Gly-Thr-Lys-
Glu-Val-Met-Thr-Pro-Pro-Thr-Lys-Thr-Ser-Trp-
Thr-Arg-Gly-Ser-Asn-Ser-Ser-Leu-Met-Arg-Phe-
Arg-Thr-Ala-Glu-Val-Thr-Lys-Gly-Gly-Arg-Val-
Ala-Gly-Val-Pro-Pro-Arg-Ser-Phe-Pro-Pro-Pro-
Cys-Gln-Arg-Lys-Ile-Glu-Ile-Asn-Lys-Thr-Phe-
Lys-Tyr-Ile-Asn-Thr-Ile-Val-Ser-Cys-Leu-Val-
Phe-Val-Leu-Gly-Ile-Ile-Gly-Asn-Ser-Thr-Leu-
Leu-Arg-Ile-Ile-Tyr-Lys-Asn-Lys-Cys-Met-Arg-
Asn-Gly-Pro-Asn-Ile-Leu-Ile-Ala-Ser-Leu-Ala-
Leu-Gly-Asp-Leu-Leu-His-Ile-Ile-Ile-Asp-Ile-
Pro-Ile-Asn-Ala-Tyr-Lys-Leu-Leu-Ala-Gly-Asp-
Trp-Pro-Phe-Gly-Ala-Glu-Met-Cys-Lys-Leu-Val-
Pro-Phe-Ile-Gln-Lys-Ala-Ser-Val-Gly-Ile-Thr-
Val-Leu-Ser-Leu-Cys-Ala-Leu-Ser-Ile-Asp-Arg-
Tyr-Arg-Ala-Val-Ala-Ser-Trp-Ser-Arg-Ile-Lys-
Gly-Ile-Gly-Val-Pro-Lys-Trp-Thr-Ala-Val-Glu-
Ile-Val-Leu-Ile-Trp-Val-Val-Ser-Val-Val-Leu-
Ala-Val-Pro-Glu-Ala-Ile-Gly-Phe-Asp-Val-Ile-

# FIG. 1B

Thr-Ser-Asp-Tyr-Lys-Gly-Lys-Pro-Leu-Arg-Val-
Cys-Met-Leu-Asn-Pro-Phe-Gln-Lys-Thr-Ala-Phe-
Met-Gln-Phe-Tyr-Lys-Thr-Ala-Lys-Asp-Trp-Trp-
Leu-Phe-Ser-Phe-Tyr-Phe-Cys-Leu-Pro-Leu-Ala-
Ile-Thr-Ala-Ile-Phe-Tyr-Thr-Leu-Met-Thr-Cys-
Glu-Met-Leu-Arg-Lys-Lys-Ser-Gly-Met-Gln-Ile-
Ala-Leu-Asn-Asp-His-Leu-Lys-Gln-Arg-Arg-Glu-
Val-Ala-Lys-Thr-Val-Phe-Cys-Leu-Val-Leu-Val-
Phe-Ala-Leu-Cys-Trp-Leu-Pro-Leu-His-Leu-Ser-
Arg-Ile-Leu-Lys-Leu-Thr-Leu-Tyr-Asp-Gln-Ser-
Asn-Pro-Gln-Arg-Cys-Glu-Leu-Leu-Ser-Phe-Leu-
Leu-Val-Leu-Asp-Tyr-Ile-Gly-Ile-Asn-Met-Ala-
Ser-Leu-Asn-Ser-Cys-Ile-Asn-Pro-Ile-Ala-Leu-
Tyr-Leu-Val-Ser-Lys-Arg-Phe-Lys-Asn-Cys-Phe-
Lys-Ser-Cys-Leu-Cys-Cys-Trp-Cys-Gln-Thr-Phe-
Glu-Glu-Lys-Gln-Ser-Leu-Glu-Glu-Lys-Gln-Ser-
Cys-Leu-Lys-Phe-Lys-Ala-Asn-Asp-His-Gly-Tyr-
Asp-Asn-Phe-Arg-Ser-Ser-Asn-Lys-Tyr-Ser-Ser-
Ser

# FIG. 2A

```
-202 GGTGGCGTGCGCCCAAGTTCCCCATTGGCGCGCAAACTTAACTTA  -158

-157 CTGTTGTGGCGCGGGTAGAGACAACCCGGCTAGGGTGAGTGTTTT  -113

-112 CAGAGGCGTGGCTGGGTAGCTGACTAAAGTACCCTCTCTTCATTC   -68

 -67 CCCTGTTGTTCTCCAGACTGAAAACGGCGGAGCGGCTACGGGACT   -23

 -22 CTCACAGGAGCAAGCTGCAACATGCAATCGTCCGCAAGCCGGTGC    22
                            MetGlnSerSerAlaSerArgCys

  23 GGACGCGCCTTGGTGGCGCTGCTGCTGGCCTGTGGCTTGTTGGGG    67
     GlyArgAlaLeuValAlaLeuLeuLeuAlaCysGlyLeuLeuGly

  68 GTATGGGGAGAGAAAAGAGGATTCCCACCTGCCCAGGCCACACCA   112
     ValTrpGlyGluLysArgGlyPheProProAlaGlnAlaThrPro

 113 TCTCTTCTCGGGACTAAAGAAGTTATGACGCCACCCACTAAGACC   157
     SerLeuLeuGlyThrLysGluValMetThrProProThrLysThr

 158 TCCTGGACTAGAGGTTCCAACTCCAGTCTGATGCGTTTCCGCACT   202
     SerTrpThrArgGlySerAsnSerSerLeuMetArgPheArgThr

 203 GCGGAGGTGACCAAAGGAGGGAGGGTGGCTGGAGTCCCGCCAAGA   247
     AlaGluValThrLysGlyGlyArgValAlaGlyValProProArg

 248 TCCTTCCCTCCTCCGTGCCAACGAAAAATTGAGATCAACAAGACT   292
     SerPheProProProCysGlnArgLysIleGluIleAsnLysThr

 293 TTTAAATACATCAACACGATTGTATCATGCCTCGTGTTCGTGCTA   337
     PheLysTyrIleAsnThrIleValSerCysLeuValPheValLeu

 338 GGCATCATCGGGAACTCCACACTGCTAAGAATCATCTACAAGAAC   382
     GlyIleIleGlyAsnSerThrLeuLeuArgIleIleTyrLysAsn

 383 AAGTGCATGAGAAATGGTCCCAATATCTTGATCGCCAGCCTGGCT   427
     LysCysMetArgAsnGlyProAsnIleLeuIleAlaSerLeuAla

 428 CTGGGAGATCTGCTACACATCATCATCGACATTCCCATTAATGCC   472
     LeuGlyAspLeuLeuHisIleIleIleAspIleProIleAsnAla

 473 TACAAGCTGCTGGCAGGGGACTGGCCATTTGGAGCTGAGATGTGC   517
     TyrLysLeuLeuAlaGlyAspTrpProPheGlyAlaGluMetCys

 518 AAGCTGGTGCCCTTCATACAGAAGGCTTCTGTGGGGATCACAGTG   562
     LysLeuValProPheIleGlnLysAlaSerValGlyIleThrVal
```

# FIG. 2B

```
 563 TTGAGTCTATGTGCTCTAAGTATTGACAGATATCGAGCTGTTGCT   607
     LeuSerLeuCysAlaLeuSerIleAspArgTyrArgAlaValAla

 608 TCTTGGAGTCGAATTAAAGGAATTGGGGTTCCAAAATGGACAGCA   652
     SerTrpSerArgIleLysGlyIleGlyValProLysTrpThrAla

 653 GTAGAAATTGTTTTAATTTGGGTGGTCTCTGTGGTTCTGGCTGTC   697
     ValGluIleValLeuIleTrpValValSerValValLeuAlaVal

 698 CCTGAAGCCATAGGTTTTGATGTGATTACGTCGGACTACAAAGGA   742
     ProGluAlaIleGlyPheAspValIleThrSerAspTyrLysGly

 743 AAGCCCCTAAGGGTCTGCATGCTTAATCCCTTTCAGAAAACAGCC   787
     LysProLeuArgValCysMetLeuAsnProPheGlnLysThrAla

 788 TTCATGCAGTTTTACAAGACAGCCAAAGACTGGTGGCTGTTCAGT   832
     PheMetGlnPheTyrLysThrAlaLysAspTrpTrpLeuPheSer

 833 TTCTACTTCTGCTTGCCGCTAGCCATCACTGCGATCTTTTACACC   877
     PheTyrPheCysLeuProLeuAlaIleThrAlaIlePheTyrThr

 878 CTAATGACCTGTGAGATGCTCAGAAAGAAAAGTGGTATGCAGATT   922
     LeuMetThrCysGluMetLeuArgLysLysSerGlyMetGlnIle

 923 GCCTTGAATGACCACTTAAAGCAGAGACGAGAAGTGGCCAAGACA   967
     AlaLeuAsnAspHisLeuLysGlnArgArgGluValAlaLysThr

 968 GTATTCTGCCTGGTCCTCGTGTTTGCCCTCTGTTGGCTTCCCCTT  1012
     ValPheCysLeuValLeuValPheAlaLeuCysTrpLeuProLeu

1013 CACCTCAGCAGGATTCTGAAGCTCACCCTTTATGACCAGAGCAAT  1057
     HisLeuSerArgIleLeuLysLeuThrLeuTyrAspGlnSerAsn

1058 CCTCAGAGGTGTGAACTTCTGAGTTTTTTGCTGGTTTTGGACTAC  1102
     ProGlnArgCysGluLeuLeuSerPheLeuLeuValLeuAspTyr

1103 ATTGGTATCAACATGGCTTCTTTGAATTCCTGCATTAATCCAATC  1147
     IleGlyIleAsnMetAlaSerLeuAsnSerCysIleAsnProIle

1148 GCTCTGTATTTGGTGAGCAAGAGATTCAAAAACTGCTTTAAGTCG  1192
     AlaLeuTyrLeuValSerLysArgPheLysAsnCysPheLysSer

1193 TGTTTGTGCTGCTGGTGCCAAACGTTTGAGGAAAAACAGTCCTTA  1237
     CysLeuCysCysTrpCysGlnThrPheGluGluLysGlnSerLeu

1238 GAGGAGAAGCAATCCTGCTTGAAGTTCAAAGCTAACGATCACGGA  1282
     GluGluLysGlnSerCysLeuLysPheLysAlaAsnAspHisGly
```

# FIG. 2C

1283 TACGACAACTTCCGCTCCAGCAATAAATACAGCTCATCTTGAAGG 1327
TyrAspAsnPheArgSerSerAsnLysTyrSerSerSer***

1328 AAGGAACACTCACTGAATCTCATTGTCCTCATCGTGGACAGATAG 1372

1373 CATTAAAACAAAATGAAACCTTTGCCAAACCCAAACGGAAAACCG 1417

1418 TGCTTGCGGAAAGGTGTGCACGCATGGGAGAGGGATTGTTTTTTA 1462

1463 ACCGTTCTAACTTTCCACACCTGATATTTCACGGGCTGTTTACAA 1507

1508 CCTAAGAAAGCCATGGGAATGAATGAAGCCTCGGGAAAGCACTTA 1552

1553 GATTCTTAGTCAAGCACTTCAGCACGGCTCTTAAAAGCCCTCACT 1597

1598 GCACTCACAGCCCACTTACATTTAAAAACAAGAACTCAAACTCTA 1642

1643 TTCAGGGGTTTATTATCCAGTCCTATGAATCTGGATACAGGAATG 1687

1688 CATGACATTGCAAAACAATTCTTAAAGCAAAGTTTCAATTGCTCG 1732

1733 ATTTGAGACAAAAAACAAAACAAAAAAAAA 1762

FIG. 3

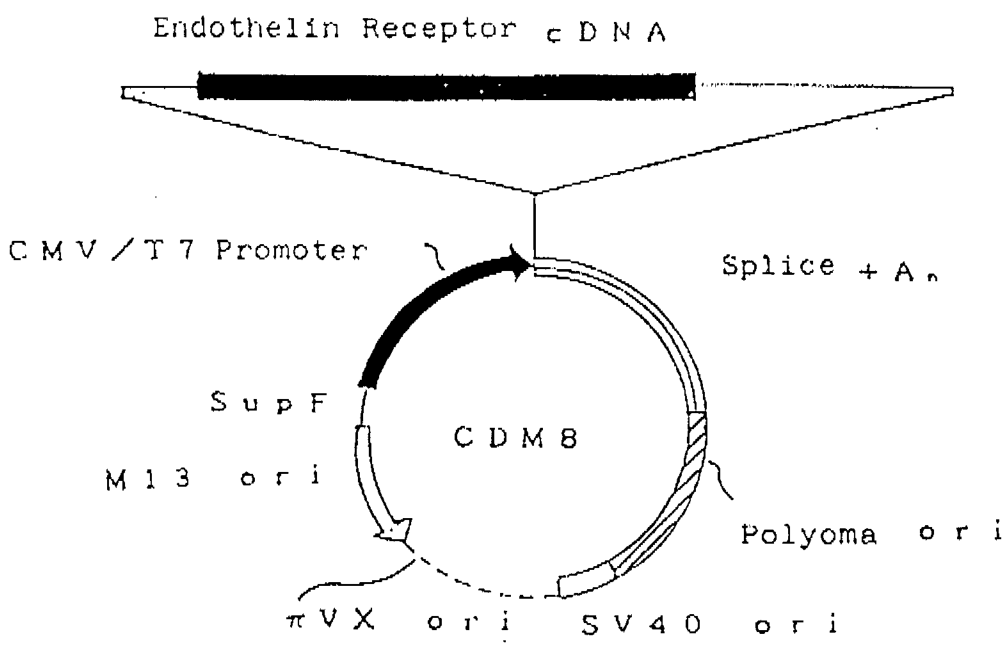
Endothelin Receptor cDNA
CMV/T7 Promoter
Splice + An
SupF
CDM8
M13 ori
Polyoma ori
πVX ori
SV40 ori

FIG. 4

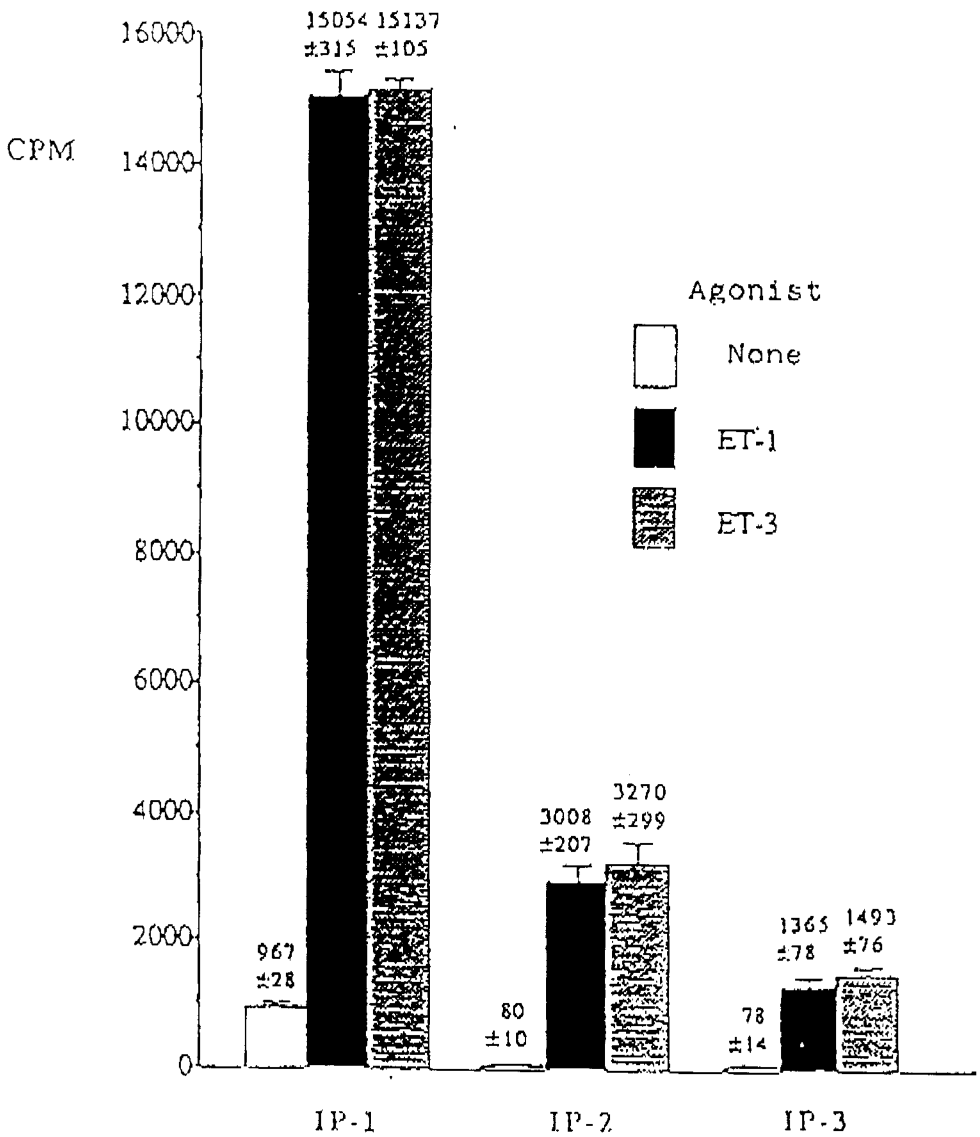
CPM
16000
14000
12000
10000
8000
6000
4000
2000
0
15054
±315
15137
±105
967
±28
3008
±207
3270
±299
80
±10
1365
±78
1493
±76
78
±14
Agonist
None
ET-1
ET-3
IP-1
IP-2
IP-3

FIG. 5

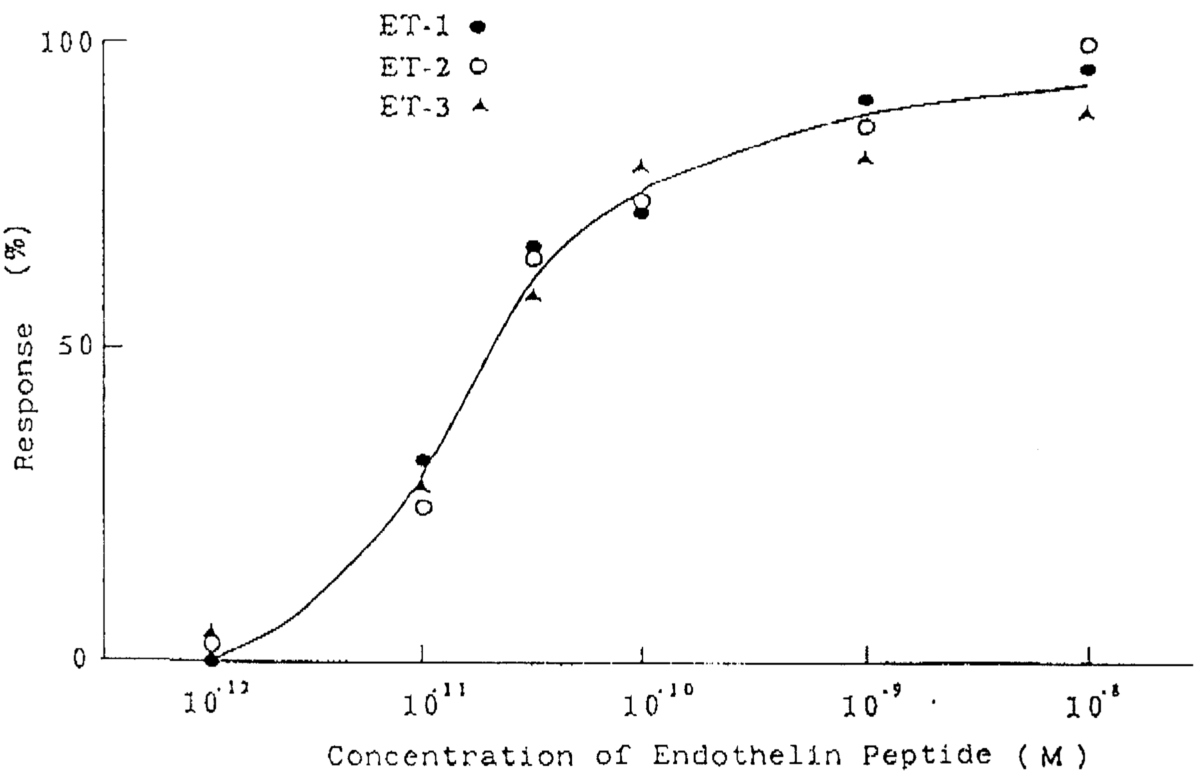
ET-1 ●
ET-2 ○
ET-3 ⅄
100
50
0
Response (%)
10⁻¹²
10⁻¹¹
10⁻¹⁰
10⁻⁹
10⁻⁸
Concentration of Endothelin Peptide (M)

FIG. 6

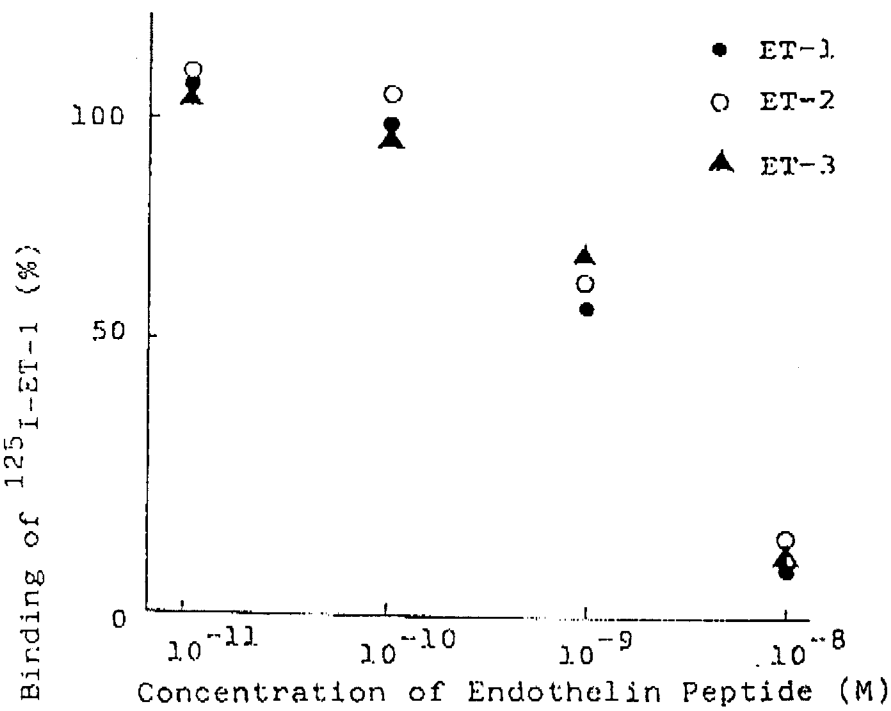
ET-1
ET-2
ET-3
100
50
0
Binding of 125I-ET-1 (%)
10-11
10-10
10-9
10-8
Concentration of Endothelin Peptide (M)

FIG. 7

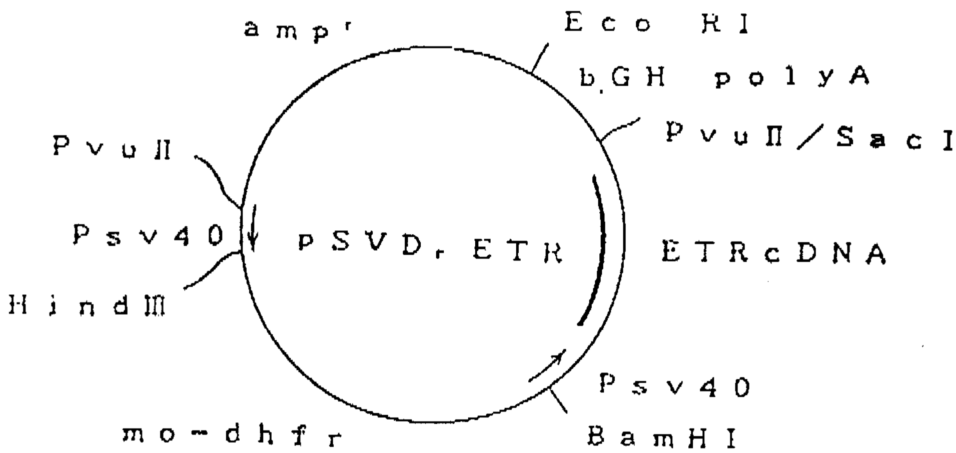
ampr
EcoRI
b.GH polyA
PvuII/SacI
PvuII
Psv40
pSVDr ETR
ETRcDNA
HindIII
Psv40
mo-dhfr
BamHI

European Patent Office

# EUROPEAN SEARCH REPORT

Application Number

**EP 91 11 7144**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | NATURE vol. 348, 20/27 December 1990, pages 732-735, London, UK; T. SAKURAI et al.: "Cloning of a cDNA encoding a non-isopeptide-selective subtype of the endothelin receptor" * whole article * | 1-14 | C 12 N 15/12<br>C 07 K 15/00<br>C 12 P 21/02<br>A 61 K 37/02<br>G 01 N 33/68 |
| P,X | PROC. NATL. ACAD. SCI. USA vol. 88, no. 8, April 1991, pages 3185-3189, Washington, DC, US; H.Y. LIN et al.: "Cloning and functional expression of a vascular smooth muscle endothelin 1 receptor" * whole article * | 1-14 | |
| P,X | NATURE vol. 348, 20/27 December 1990, pages 730-732, London, UK; H. AKAI et al.: "Cloning and expression of a cDNA-encoding an endothelin receptor" * whole article * | 1-14 | |
| P,X | BIOCHEM. BIOPHYS. RESEARCH COMMUNICATIONS vol. 177, no. 1, 31 May 1991, pages 34-39, New York, US; M NAKAMUTA et al.: "Cloning and sequence analysis of a cDNA encoding human non-selective type of endothelin receptor" * whole article * | 1-14 | **TECHNICAL FIELDS SEARCHED (Int. Cl.5)** |
| A | BIOCHEM. BIOPHYS. RESEARCH COMMUNICATIONS vol. 167, no. 1, 28 February 1990, pages 251-257, New York, US; K. WADA et al.: "Purification of a endothelin receptor from human placenta" * whole article * | 1,13,14 | C 12 N 15/12<br>C 07 K 15/00 |
| A | CHEMICAL ABSTRACTS vol. 113, no. 1, 2 July 1990, abstract no. 1064n, Columbus, Ohio, US; I. SCHVARTZ et al.: "Identification of endothelin receptors by chemical cross-linking" & Endocrinology 1990, vol 126, no. 4, pages 1829-1833 * abstract * | 1,13,14 | |

The present search report has been drawn up for all claims

| Place of search | Date of completion of search | Examiner |
|---|---|---|
| Berlin | 02 December 91 | JULIA P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document